# EUROPEAN PATENT SPECIFICATION

(11) **EP 4 111 956 B1**
(45) Date of publication and mention of the grant of the patent: **25.02.2026**
(21) Application number: 22175447.6
(22) Date of filing: 25.05.2022
(51) Int. Cl.: A61B 5/0537, A61B 5/145, A61B 5/1477

(54) **APPARATUS AND METHOD FOR ESTIMATING BIO-INFORMATION BASED ON BIO-IMPEDANCE**
GERÄT UND VERFAHREN ZUR SCHÄTZUNG VON BIOINFORMATIONEN BASIEREND AUF BIOIMPEDANZ
APPAREIL ET PROCÉDÉ D'ESTIMATION D'INFORMATIONS BIOLOGIQUES SUR LA BASE DE BIO-IMPÉDANCE

(30) Priority: 02.07.2021 KR 20210087354; 21.12.2021 KR 20210183603
(43) Date of publication of application: 04.01.2023
(73) Proprietor: Samsung Electronics Co., Ltd., Suwon-si, Gyeonggi-do 16677 (KR)
(72) Inventor: Lee, Yeol Ho, Uiwang-si (KR); Lee, Joon Hyung, Seongnam-si (KR); Kwon, Yong Joo, Yongin-si (KR); Park, Yun S, Suwon-si (KR)
(74) Representative: Grünecker Patent- und Rechtsanwälte PartG mbB

(56) References cited:
- WO-A1-2017/116358
- US-A1- 2004 059 242
- US-A1- 2019 167 208
- US-A1- 2020 323 468

## Description

### BACKGROUND

### 1. Field

Apparatuses and methods consistent with example embodiments relate to estimating bio-information by measuring bio-impedance in a non-invasive manner.

### 2. Description of the Related Art

Various medical devices are being developed for diagnosing patients' health conditions. For patients' convenience and rapid health diagnosis results during health diagnosis, great importance is placed on medical devices for measuring bioelectric signals from patients. Particularly, bio-impedance may be used for monitoring the health of a living body and emotional states, and various studies are underway to provide devices for measuring such bio-impedance in a compact size and to provide a method of measuring the bio-impedance rapidly and accurately.
US 2020/323468 A1 discloses an apparatus for analyzing an in vivo component. The apparatus includes an impedance sensor comprising a first electrode and a second electrode configured to contact a fluid channel of a fluid to be analyzed. The apparatus further includes an impedance measurement device configured to apply a current to the first electrode and the second electrode, measure a voltage between the first electrode and the second electrode based on applying the current, and measure an impedance of the fluid based on the measured voltage.
US 2019/167208 A1 discloses a bio-information processing apparatus including: a main body; a strap which is connected to both ends of the main body; an impedance measurer configured to measure a bio-impedance of a user while the main body and the strap are in contact with the user; and a processor configured to estimate a body water amount of the user by applying the measured bio-impedance to a body water estimation model.
WO 2017/116358 A1 discloses an apparatus for determining free fatty acids and/or fatty acid binding capacity of albumin in case of acute organ/tissue ischemia, or in acute/chronic specific medical conditions associated with modified albumin by fatty acids, and diagnosing medical conditions associated with increased free fatty acids and/or reduced fatty acid binding capacity of albumin in samples, using electrical impedance measurement.
US 2004/059242 A1 discloses a body composition measurement method and system. The method comprises determining four distal voltage-measuring points Pv at the wrists and ankles, and four proximal voltage-measuring points Pv at the elbows and knees. Four current-carrying electrodes and four measuring electrodes are used. First, the measuring electrodes are attached to distal points, and the impedances of four limbs and the trunk are measured. Then, the measuring electrodes are moved to proximal points, and the impedances of four limbs and the trunk are measured.
It is the object of the present invention to improve the estimation accuracy of bio-information.
This object is solved by the subject matter of the independent claims.
Embodiments are defined in the dependent claims.

The invention is claimed in the independent claims. Preferred embodiments are specified in the dependent claims.

According to an aspect of the disclosure, there is provided an apparatus for estimating bio-information, including: an impedance sensor including a pair of input electrodes and a pair of receiving electrodes, and configured to measure bio-impedance of a user by applying a current to the pair of input electrodes and by measuring a voltage between the pair of receiving electrodes; and a processor configured to estimate bio-information by applying, to the measured bio-impedance, an estimation model that uses a correlation between the measured bio-impedance and the bio-information to be estimated, wherein the bio-information includes at least one of free fatty acid, triglyceride, and a blood test marker.

The correlation may include a correlation between the bio-impedance and a free fatty acid concentration in a tissue fluid. The processor may be further configured to generate the estimation model based on the correlation between the free fatty acid concentration in the tissue fluid and the free fatty acid concentration in blood, and estimate the free fatty acid concentration in blood from the free fatty acid concentration in the tissue fluid.

The bio-information may include at least one of an accumulated fat level, a hydrolyzed fat level, a consumed fat level, and calories that have turned into energy. Based on the correlation including a correlation between the free fatty acid concentration in blood and the at least one of the accumulated fat level, the hydrolyzed fat level, the consumed fat level, and the calories that have turned into energy, the processor may be further configured to obtain at least one of the accumulated fat level, the hydrolyzed fat level, the consumed fat level, and the calories that have turned into energy from the estimated free fatty acid concentration in blood.

The blood test marker includes at least one of uric acid, creatinine, cholesterol, blood pressure, glycated hemoglobin (HbA1C), blood glucose, hematocrit (Hct), and plasma protein.

The processor may be further configured to generate at least one of exercise information or food intake information for guiding the user, based on an estimated triglyceride concentration or the blood test marker.

The processor may be further configured to generate the estimation model that uses regression analysis to estimate the blood test marker, based on at least one of a gender, an age, a stature, and a weight of the user.

The processor may be further configured to generate user guidance information about a bio-impedance measurement position according to a type of the bio-information to be estimated.

In response to the bio-information to be estimated being the free fatty acid or the triglyceride, the processor may be further configured to control the impedance sensor to measure an abdomen impedance from a stomach of the user; and in response to the bio-information to be estimated being the blood test marker, the processor may be further configured to control the impedance sensor to measure a partial impedance from a torso, an arm, or a leg of the user.

The impedance sensor may be further configured to measure the partial impedance from the torso, the arm, or the leg of the user, and the processor may be further configured to transform the partial impedance into the abdomen impedance by using a transformation model, and estimate the free fatty acid or the triglyceride based on the transformed abdomen impedance.

The apparatus may further include an output interface configured to output information on an estimation time of the bio-information by using at least one of a display, an audio output device, and a haptic device.

The output interface may include the display configured to display a change in the bio-information over a predetermined period of time.

The apparatus may further include an optical sensor configured to emit light onto an object and to detect an optical signal scattered or reflected from the object, and the processor may be further configured to generate the estimation model based on the bio-impedance and the detected optical signal.

According to another aspect of the disclosure, there is provided a method of estimating bio-information, the method including: measuring bio-impedance of a user by using an impedance sensor; and estimating bio-information by applying, to the measured bio-impedance, an estimation model that uses a correlation between the measured bio-impedance and the bio-information to be estimated, wherein the bio-information may include at least one of free fatty acid, triglyceride, and a blood test marker.

The correlation may include a correlation between the bio-impedance and a free fatty acid concentration in a tissue fluid, and wherein the estimating of the bio-information may include: estimating the free fatty acid concentration in the tissue fluid by using the estimation model based on the correlation between the bio-impedance and the free fatty acid concentration in the tissue fluid; and estimating the free fatty acid concentration in blood from the free fatty acid concentration in the tissue fluid based on a correlation between the free fatty acid concentration in the tissue fluid and the free fatty acid concentration in blood.

The bio-information may further include at least one of an accumulated fat level, a hydrolyzed fat level, a consumed fat level, and calories that have turned into energy. The method may further include, based on a correlation between a free fatty acid concentration and the at least one of the accumulated fat level, the hydrolyzed fat level, the consumed fat level, and the calories that have turned into energy, obtaining at least one of the accumulated fat level, the hydrolyzed fat level, the consumed fat level, and the calories that have turned into energy from the estimated free fatty acid concentration in blood.

The method may further include generating user guidance information about a bio-impedance measurement position according to a type of the bio-information to be estimated.

The method may further include: when the impedance sensor measures a partial impedance from a torso, an arm, or a leg of the user, transforming the measured partial impedance into an abdomen impedance by using a transformation model, and generating the estimation model for estimating the free fatty acid or the triglyceride based on the transformed abdomen impedance.

According to another aspect of the disclosure, there is provided an apparatus for estimating bio-information, including: an impedance sensor including a pair of input electrodes and a pair of receiving electrodes, and configured to measure bio-impedance of a user by applying a current to the pair of input electrodes and by measuring a voltage between the pair of receiving electrodes; and a processor configured to obtain at least one of an accumulated fat level, a hydrolyzed fat level, a consumed fat level, and calories that have turned into energy based on the measured bio-impedance.

The processor may be further configured to generate an estimation model by analyzing a correlation between the bio-impedance and the at least one of the accumulated fat level, the hydrolyzed fat level, the consumed fat level, and the calories that have turned into energy, and obtains at least one of the accumulated fat level, the hydrolyzed fat level, the consumed fat level, and the calorie level that have turned into energy from the bio-impedance by using the generated estimation model.

According to another aspect of the disclosure, there is provided an electronic device including: a main body; an impedance sensor including a first electrode pair disposed on a first surface of the main body to come into contact with a first body part of a user, a second electrode pair disposed on second surface of the main body to come into contact with a second body part of the user, and a measurer configured to measure bio-impedance using the first electrode pair and the second electrode pair; and a processor configured to estimate bio-information by using an estimation model that applies a correlation between the measured bio-impedance and the bio-information, to the measured bio-impedance, wherein the bio-information may include at least one of free fatty acid, triglyceride, and a blood test marker.

### BRIEF DESCRIPTION OF THE DRAWINGS

The above and/or other aspects will be more apparent by describing certain example embodiments, with reference to the accompanying drawings, in which:
FIG. 1 is a block diagram illustrating an apparatus for estimating bio-information according to an example embodiment of the present disclosure;
FIG. 2A is a diagram illustrating blood fatty acid levels of three subjects which are measured in a blood test;
FIGS. 2B and 2C are diagrams explaining a correlation between bio-impedance and free fatty acid;
FIGS. 3A and 3B are diagrams explaining a correlation between bio-impedance and triglyceride;
FIGS. 4A and 4B are diagrams explaining a correlation between bio-impedance and uric acid or creatinine;
FIG. 5 is a block diagram illustrating an apparatus for estimating bio-information according to another example embodiment of the present disclosure;
FIGS. 6A and 6B are diagrams illustrating examples of providing a bio-information estimation result to a user;
FIG. 7 is a block diagram illustrating an apparatus for estimating bio-information according to yet another example embodiment of the present disclosure;
FIGS. 8 to 12 are flowcharts illustrating a method of estimating bio-information according to embodiments of the present disclosure; and
FIGS. 13 to 17 are diagrams schematically illustrating structures of various electronic devices including an apparatus for estimating bio-information.

### DETAILED DESCRIPTION

Example embodiments are described in greater detail below with reference to the accompanying drawings.

In the following description, like drawing reference numerals are used for like elements, even in different drawings. The matters defined in the description, such as detailed construction and elements, are provided to assist in a comprehensive understanding of the example embodiments. However, it is apparent that the example embodiments can be practiced without those specifically defined matters. Also, well-known functions or constructions are not described in detail since they would obscure the description with unnecessary detail.

It will be understood that, although the terms first, second, etc. may be used herein to describe various elements, these elements should not be limited by these terms. These terms are only used to distinguish one element from another. Also, the singular forms are intended to include the plural forms as well, unless the context clearly indicates otherwise. It will be further understood that when an element is referred to as "comprising" another element, the element is intended not to exclude one or more other elements, but to further include one or more other elements, unless explicitly described to the contrary. In the following description, terms such as "unit" and "module" indicate a unit for processing at least one function or operation and they may be implemented by using hardware, software, or a combination thereof.

Expressions such as "at least one of," when preceding a list of elements, modify the entire list of elements and do not modify the individual elements of the list. For example, the expression, "at least one of a, b, and c," should be understood as including only a, only b, only c, both a and b, both a and c, both b and c, all of a, b, and c, or any variations of the aforementioned examples.

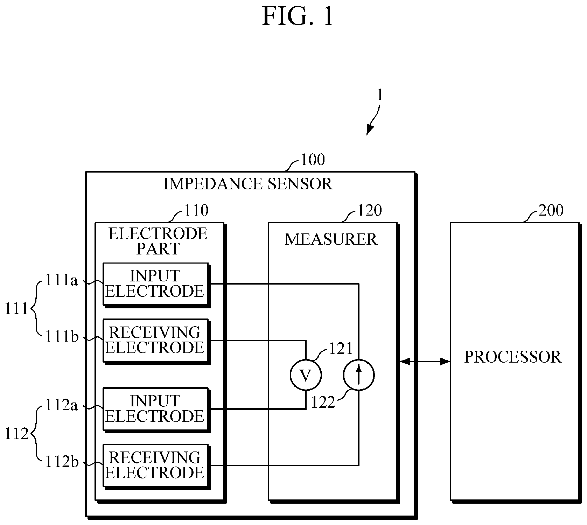

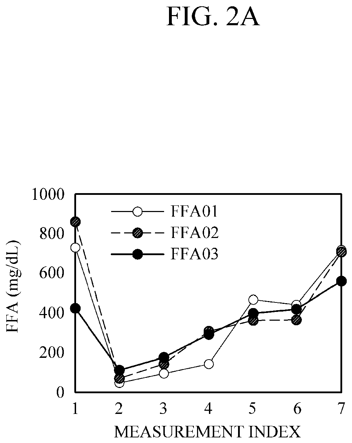

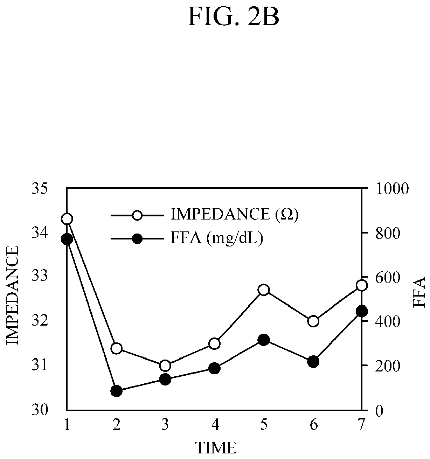

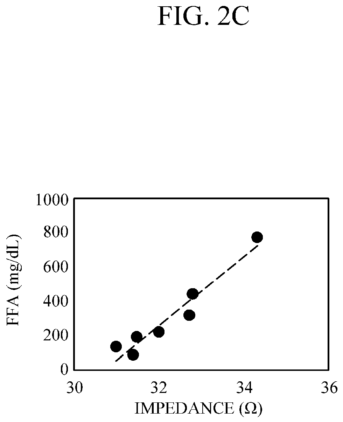

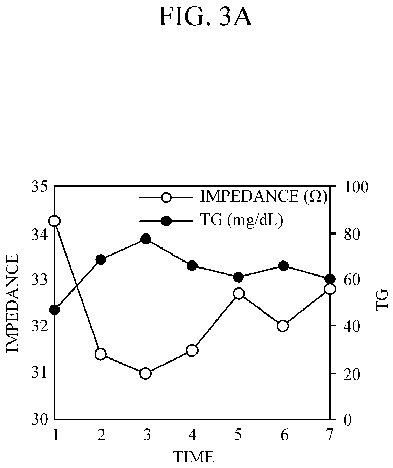

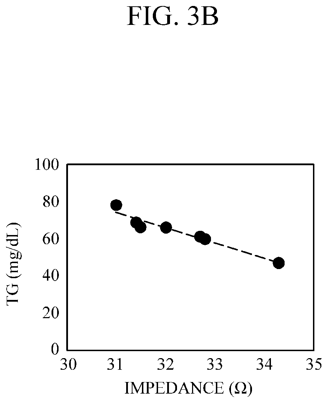

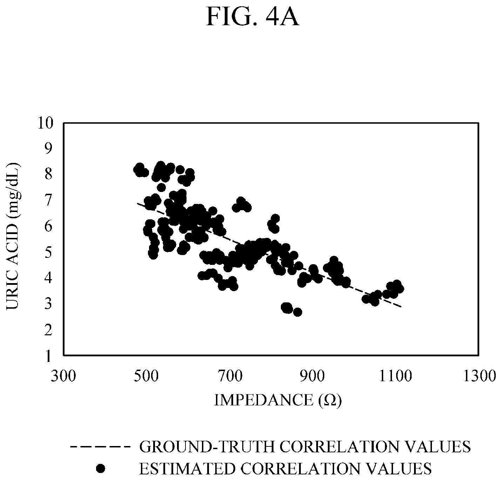

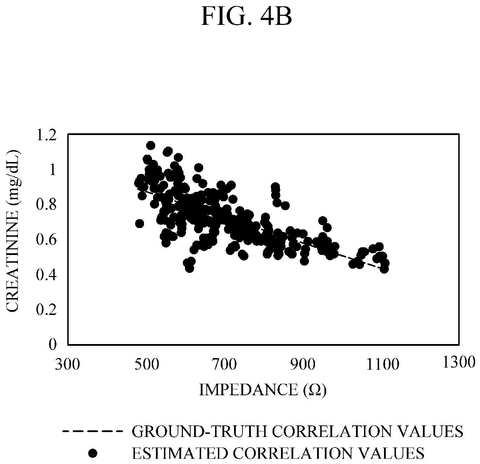

FIG. 1 is a block diagram illustrating an apparatus for estimating bio-information according to an example embodiment of the present disclosure. FIG. 2A is a diagram illustrating blood fatty acid levels of three subjects which are measured in a blood test. FIGS. 2B and 2C are diagrams explaining a correlation between bio-impedance and free fatty acid. FIGS. 3A and 3B are diagrams explaining a correlation between bio-impedance and triglyceride. FIGS. 4A and 4B are diagrams explaining a correlation between bio-impedance and uric acid or creatinine.

Referring to FIG. 1, an apparatus 1 for estimating bio-information includes an impedance sensor 100 and a processor 200. The components 100 and 200 of the apparatus 1 for estimating bio-information may be integrally formed in a single hardware device, or the impedance sensor 100 and the processor 200 may be formed in separate hardware devices to be electrically connected to each other directly or through wireless communications.

The impedance sensor 100 may include an electrode part 110 and a measurer 120, and may measure bio-impedance from a user.

The electrode part 110 may be composed of a first electrode part 111 and a second electrode part 112. As illustrated herein, the respective electrode parts 111 and 112 may measure the bio-impedance by a four-electrode method with input electrodes 111a and 112a and receiving electrodes 111b and 112b. The input electrodes 111a and 112a and the receiving electrodes 111b and 112b may be referred to as current electrodes 111a and 112a and voltage electrodes 111b and 112b, respectively. However, the electrode parts are not limited thereto, and the respective electrode parts 111 and 112 may be formed as a single electrode to measure the bio-impedance by a two-electrode method. The input electrodes 111a and 112a and the receiving electrodes 111b and 112b may have various shapes such as a bar shape, a rectangular U shape, a semicircular shape, a circular shape, and the like.

The measurer 120 may apply a current to the input electrodes 111a and 112a of the first electrode part 111 and the second electrode part 112 when the first electrode part 111 and the second electrode part 112 are in contact with a user's body part. While applying the current to the input electrodes 111a and 112a, the measurer 120 may measure a voltage between the receiving electrodes 111b and 112b, so that the processor 200 may determine a body impedance of the user based on the value of the current applied to the input electrodes 111a and 112a, and the value of the voltage detected from the receiving electrodes 111b and 112b. For example, the current may be a constant current of 500 µA at a frequency of 50 kHz. As illustrated herein, the measurer 120 may include a voltmeter 121 for measuring a voltage and a current source 122 for applying an alternating current. The measurer 120 may obtain impedance spectrum data by measuring a plurality of impedances while changing the frequency of the input current in a predetermined frequency band (e.g., frequency band of 1 kHz to hundreds of MHz). However, the measurer 120 is not limited thereto, and may measure the bio-impedance by applying a constant voltage to the input electrodes 111a and 112a and by measuring a voltage between the receiving electrodes 111b and 112b. The measurer 120 may use a battery in the apparatus 1 for estimating bio-information as a current source or may use power of an external device connected by wire or wirelessly to the apparatus 1.

The processor 200 may generate an estimation model by analyzing a correlation between the bio-impedance, measured from the user's body part, and a variety of bio-information to be estimated. The processor 200 may estimate bio-information based on the bio-impedance by using the generated estimation model. In particular, by further using user characteristic information including at least one of a user's gender, age, stature, and weight in addition to the bio-impedance, the processor 200 may generate an estimation model personalized for each user. The bio-information estimation model may be expressed as a linear function, but is not limited thereto and may be defined as various nonlinear functions such as a logarithmic function and the like.

For example, the processor 200 may generate a free fatty acid estimation model by analyzing a correlation between bio-impedance and free fatty acid, and may obtain a free fatty acid concentration based on the bio-impedance by using the generated free fatty acid estimation model.

FIG. 2A is a diagram illustrating blood fatty acid levels of three subjects FFA01, FFA02, and FFA03 which are measured in a blood test, in which the X axis denotes a time domain including a plurality of measurement time indexes, and the Y axis denotes free fatty acid (FFA) levels in blood at each measurement time. A first measurement (i.e., measurement index 1 in FIG. 2A) shows fatty acid levels measured in a fasting state after fasting for 12 hours or more, and a second measurement (i.e., measurement index 2 in FIG. 2A) shows fatty acid levels measured one hour after food intake when the first measurement is complete. The measurements from the second measurement to a seventh measurement show fatty acid levels measured at one hour intervals without food intake. It can be seen that the fatty acid levels measured after fasting for 12 hours or more are much greater than the second test performed after a meal, and immediately after the meal, the fatty acid levels decrease sharply, and then increase again three hours after the meal.

Generally, when the body lacks energy after fasting or skipping a meal, or when the body requires energy after exercise and the like, the triglyceride (TG) breaks down into fatty acids and glycerol in the mitochondria of fat cells. The triglyceride (TG) is composed of one molecule of glycerol and three molecules of fatty acids. In this case, the hydrolyzed fatty acids, which are also referred to as free fatty acids, are hydrolyzed in fat cells to pass through blood vessels, and then are transported to each body tissue, e.g., muscle and the like, which requires energy for use in generating an energy source. The transported free fatty acids are oxidized in the mitochondria to create adenosine triphosphate (ATP) as a final source of energy.

The free fatty acids are electrically nonpolar and hydrophobic, and the free fatty acids transported to the body tissue are also present in the interstitial tissue fluid or the interstitial fluid while the free fatty acids are transported to tissue cells. The fatty acid concentration and the fatty acid variation in the tissue fluid are almost equal to the fatty acid concentration and the fatty acid variation in blood.

Based on a relationship between the free fatty acid concentration in the tissue fluid and the free fatty acid concentration in blood, and the measurement value of the bio-impedance, the processor 200 may obtain the free fatty acid concentration in blood. For example, by analyzing a correlation between the bio-impedance and the free fatty acid concentration in the tissue fluid, the processor 200 may generate an estimation model for estimating the free fatty acid concentration in the tissue fluid, and may obtain the free fatty acid concentration in blood based on the estimated free fatty acid concentration in the tissue fluid. Alternatively, by analyzing a correlation between the bio-impedance, the free fatty acid concentration in the tissue fluid, and the free fatty acid concentration in blood, the processor 200 may generate a model for estimating the free fatty acid concentration in blood directly from the bio-impedance, and may obtain the free fatty acid concentration in blood directly from the bio-impedance by using the generated model.

Generally, in bio-impedance measurement, a difference between an amount of tissue water (body water) and electrical conductivity for compositions is output as an electrical resistance value. The tissue fluid is composed of ionized electrolyte, organic acid, etc., and is maintained at a constant concentration by homeostasis. Tissue fluid composition is polar and hydrophilic, such that there is almost no change in electrical resistance according to the type of composition. There may be a difference in electrical resistance of the tissue fluid between individual users according to each individual's characteristics (e.g., obesity, muscularity, etc.), but the electrical resistance of the tissue fluid of each individual does not change abruptly in a short period due to homeostasis. The free fatty acids hydrolyzed in fat cells and transported to body tissue/tissue fluid are nonpolar and hydrophobic, such that the electrical resistance increases with an increase in free fatty acid concentration. Accordingly, while there is almost no change in electrical resistance of the tissue fluid due to homeostasis, the fatty acid is a principal substance that causes a change in electrical resistance of the tissue fluid.

FIG. 2B is a diagram illustrating a change in bio-impedance according to a change in fatty acid concentration in the tissue fluid, in which the X axis denotes a time domain including a plurality of measurement time indexes. FIG. 2C is a diagram illustrating a correlation between bio-impedance and free fatty acid. As can be seen in FIGS. 2B and 2C, a change in fatty acid concentration may be a main cause of a change in electrical resistance of the tissue fluid, such that there is a high correlation between a change in fatty acid concentration and a change in bio-impedance. As the free fatty acid is a main cause of the change in electrical resistance of the tissue fluid, and there is a high correlation between the fatty acid in the tissue fluid and the fatty acid in blood, such that by measuring a free fatty acid concentration and/or a free fatty acid variation in the tissue fluid based on bio-impedance, the free fatty acid concentration and/or the free fatty acid variation in blood may be measured.

In another example, based on a correlation between the free fatty acid concentration, a fat level (e.g., an amount of an accumulated fat, a hydrolyzed fat, a consumed fat, or fat cells), or accumulated or consumed calories, and calories that turn into energy (hereinafter "burned calories"), the processor 200 may obtain the fat level or the accumulated or consumed calories in real time or during a predetermined period of time. The term "consumed calories" may refer to the calories that have been consumed by a body. The term "accumulated calories" may refer to the calories that remain in the body after the body burns some (or all) of the consumed calories. That is, when the body requires energy after fasting/skipping a meal, exercise, etc., triglycerides are hydrolyzed in the fat cells to use fatty acids as a source of energy, such that by measuring the free fatty acid concentration or the free fatty acid variation in real time or during a predetermined period time, the processor 200 may obtain the fat level in real time or during a predetermined period of time.

For example, by using a free fatty acid estimation model that defines a correlation between the bio-impedance and the free fatty acid concentration, the processor 200 may first estimate the free fatty acid concentration, and may obtain the fat level based on the estimated free fatty acid concentration. Alternatively, based on a correlation between the bio-impedance, the free fatty acid concentration, and the fat level or the burned calories, the processor 200 may generate an estimation model for obtaining the fat level or the accumulated or consumed calories based on the bio-impedance, and may obtain the fat level or the accumulated or consumed calories directly from the bio-impedance.

In another example, the processor 200 may estimate a triglyceride concentration based on the bio-impedance by using a correlation between the free fatty acid concentration and the triglycerides. Generally, the free fatty acid is hydrolyzed from triglycerides in fat cells, and the triglyceride is composed of one molecule of glycerol and three molecules of fatty acids, such that the processor 200 may estimate the triglyceride concentration based on the free fatty acid concentration.

FIG. 3A is a diagram illustrating a change in impedance according to a change in triglyceride, and FIG. 3B is a diagram illustrating a correlation between triglyceride and impedance. If there is a large amount of hydrolyzed fatty acids, the amount of triglycerides used increases, and the amount of triglycerides in tissue decreases, such that the fatty acids and the triglycerides have a negative correlation, and the bio-impedance and the triglycerides have a negative correlation.

By using the correlation, the processor 200 may estimate a free fatty acid concentration based on the bio-impedance, and may predict a triglyceride concentration from the estimated free fatty acid concentration. Alternatively, the processor 200 may obtain the triglyceride directly from the bio-impedance by generating a triglyceride estimation model that indicates a correlation between the bio-impedance and the triglyceride concentration, and by using the generated estimation model.

In another example, the processor 200 may estimate various blood test markers, such as uric acid, creatinine, cholesterol, blood pressure, glycated hemoglobin (HbA1C), blood glucose, hematocrit (Hct), plasma protein, and the like. FIGS. 4A and 4B are diagrams respectively illustrating a correlation between bio-impedance and uric acid and a correlation between bio-impedance and creatinine. The processor 200 may generate an estimation model by analyzing a correlation between bio-impedance and a blood test marker by using regression analysis and the like, and may estimate a blood test marker by using the generated estimation model. In this case, by further using user characteristic information including at least one of a user's gender, age, stature, weight, etc., the processor 200 may generate the estimation model for estimating the blood test marker by regression analysis of the information.

In addition, by using a correlation between each bio-information to be estimated and bio-impedance measured from a body part that is highly correlated with each bio-information to be estimated, the processor 200 may generate an estimation model for each bio-information, and may estimate each bio-information by using the estimation model. Generally, the breakdown of fatty acids actively occurs in the stomach having a small amount of muscle and a large amount of fat tissue, such that the free fatty acids and triglycerides have a high correlation with an abdomen impedance, and uric acid and creatinine have a high correlation with impedance of a highly muscular body part, such as an upper body (e.g., a torso), an arm, a leg, and the like. Accordingly, the processor 200 may estimate free fatty acids or triglycerides by using the abdomen impedance, and may estimate uric acid or creatinine by using the partial impedance of the body part.

For example, if the impedance sensor 100 is configured to measure a whole body impedance or an abdomen impedance, the processor 200 may generate an estimation model for estimating free fatty acids or triglycerides by using the abdomen impedance, and may estimate the free fatty acids or triglycerides. However, the processor 200 is not limited thereto and may transform the measured abdomen impedance into a required body part impedance (e.g., an impedance of the upper body, the arm, the leg and the like) by using a predetermined transformation model and may estimate the free fatty acids or triglycerides by using the transformed body part impedance.

In another example, if the impedance sensor 100 is not capable of measuring the abdomen impedance and thus is configured to measure a partial impedance of the upper body, the arm, the leg and the like, the processor 200 may transform the partial impedance into the abdomen impedance by using a predetermined transformation model, and may estimate the free fatty acids or triglycerides by using the transformed abdomen impedance.

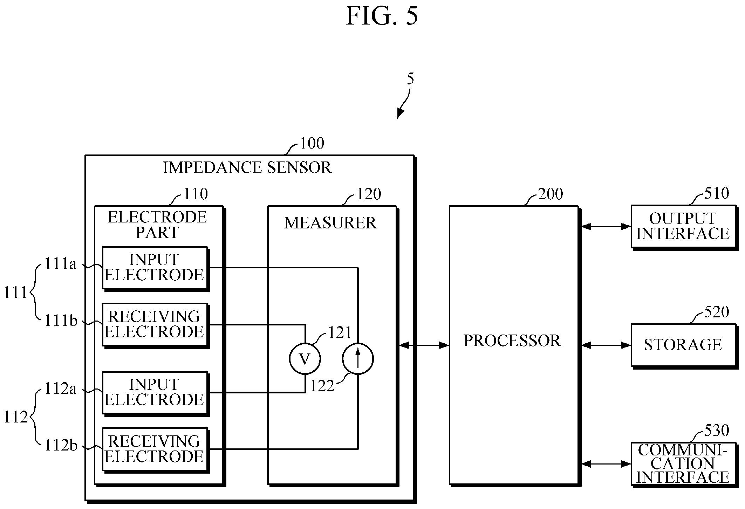

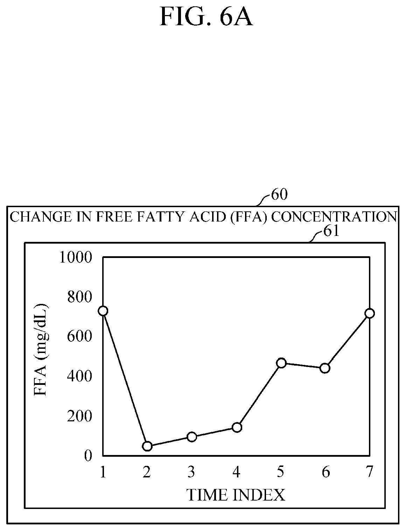

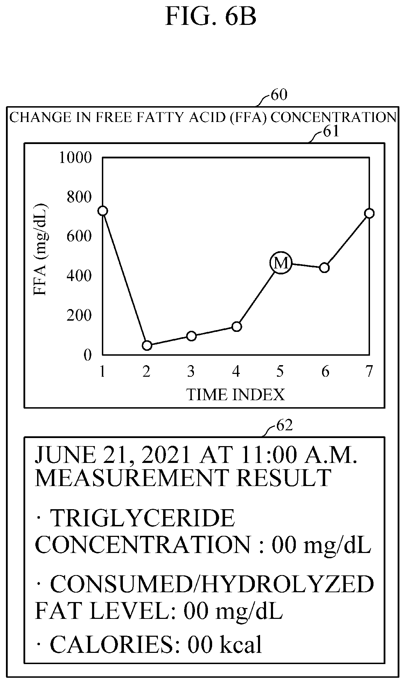

In yet another example, if the impedance sensor 100 is configured to measure a partial impedance of the upper body, the arm, the leg and the like, the processor 200 may estimate a blood test marker, such as uric acid or creatinine, by using the partial impedance of the upper body/arm/leg and the like. However, the processor 200 is not limited thereto, and may transform the partial impedance into the abdomen impedance by using the predetermined transformation model and may estimate the blood test marker, such as uric acid or creatinine, by using the transformed abdomen impedance. FIG. 5 is a block diagram illustrating an apparatus for estimating bio-information according to another example embodiment of the present disclosure. FIGS. 6A and 6B are diagrams illustrating examples of providing a bio-information estimation result to a user.

Referring to FIG. 5, an apparatus 5 for estimating bio-information may include the impedance sensor 100, the processor 200, an output interface 510, a storage 520, and a communication interface 530. The impedance sensor 100 may include: the electrode part 110 including the first electrode part 111 having a pair of the input electrode 111a and the receiving electrode 111b, and the second electrode part 112 having a pair of the input electrode 112a and the receiving electrode 112b; and the measurer 120 including the voltmeter 121 and the current source 122. The impedance sensor 100 is described in detail above, and thus a description thereof will be omitted below.

At a predetermined calibration time or in response to a user's request, or upon determining that calibration is required based on analysis of a bio-information estimation result, the processor 200 may generate an estimation model, which is personalized for a user, for each bio-information to be estimated. Further, the processor 200 may store the generated estimation model for each bio-information in the storage 520. For example, the processor 200 may measure the bio-impedance a plurality of number of times during a predetermined period of time and may generate the estimation model for each bio-information by analyzing a correlation between the measured bio-impedance at each measurement time and reference bio-information measured at each time in a blood test.

The processor 200 may guide a user on each measurement time for calibration. For example, as illustrated in FIG. 2A, the processor 200 may guide the user to measure impedance when a predetermined period of time elapses after maintaining a fasting state for a predetermined period of time. Then, after food intake, the processor 200 may guide the user to measure impedance a plurality of number of times, for example, at one-hour intervals. The output interface 510 may display guide information on a display, and may provide the user with the guide information by voice and/or alarm using an audio output device or by vibrations, tactile sensation, and the like using a haptic module at each measurement time.

In addition, upon receiving a request for estimating bio-information from a user, or at a predetermined bio-information estimation time, the processor 200 may control the impedance sensor 110 to measure bio-impedance, and may estimate bio-information by using the measured bio-impedance and a bio-information estimation model.

By referring to the storage 520, the processor 200 may guide a user on a bio-information estimation time through the output interface 510. Under the control of the processor 200, the output interface 510 may output guide information on the measurement time by using the display, the audio output device, and/or the haptic device.

For example, in order to monitor a bio-information change over a predetermined time period (e.g., day, week, month, etc.) in a fasting state, information such as a fasting time (e.g., 6 a.m.), a monitoring period, and/or intervals, etc., may be preset in the storage 520 for each user. The processor 200 may read the guide information from the storage 520 and may provide the guide information for the user at a corresponding time through the output interface 510.

In another example, in order to monitor changes, such as a change in free fatty acid concentrations, a change in free fatty acid triglyceride concentrations, a change in an amount of hydrolyzed fat (in blood), a change in an amount of consumed fat (in blood), a change in accumulated calories, a change in consumed calories, information such as food intake time, type of food, amount of food intake, exercise time, exercise period, type of exercise, a monitoring period and/or intervals may be preset in the storage 520. By referring to the storage 520, the processor 200 may guide a user on food intake, exercise, and the like at a corresponding time through the output interface 510, and after food intake or exercise, the processor 200 may guide the user to immediately measure impedance.

In yet another example, in order to monitor a bio-information change during a day, information such as a measurement time or intervals, for example, a fasting time (e.g., 6 a.m.) and predetermined time intervals (e.g., one-hour intervals, two-hour intervals, etc.) after the fasting time may be preset. By referring to the storage 520, the processor 200 may guide a user to measure impedance at a corresponding time through the output interface 510. Accordingly, by using bio-information, measured at the fasting time, as a reference, the user may monitor a change in bio-information values measured thereafter.

Once the bio-impedance is measured as described above, the processor 200 may refer to the storage 520 to estimate bio-information from the bio-impedance based on an estimation model for estimating bio-information to be estimated, and may provide an estimation result to the user through the output interface 510.

For example, the output interface 510 may display, for example, an estimated bio-information value, warning or alarm information, a change in estimated bio-information values, and the like. Alternatively, the output interface 510 may provide the estimated bio-information value, warning or alarm information, and the like for the user in a non-visual manner by voice, vibrations, tactile sensation, and the like using an audio output module, a haptic module, and the like. Further, the output interface 510 may provide recommendations, such as exercise or food suitable for a user, based on the estimated bio-information value, such as a triglyceride level or levels of hydrolyzed or consumed fat, or a change in the estimated bio-information value.

FIG. 6A is a diagram illustrating an example of outputting a change in free fatty acid concentration in a graph. As illustrated herein, the change in free fatty acid concentration during a predetermined time period (e.g., day) may be displayed in a graph 61 on a display 60. **In** particular, in order to provide a screen showing data or a graph corresponding to a previous or subsequent time point, the display 60 may output a graphic object (e.g., an arrow on the left or right side of a graph, a front or rear button on a lower end, etc.), and when a user selects a certain time point in the graph using the graphic object, the display 60 may display data corresponding to the select time point.

FIG. 6B is a diagram illustrating an example of displaying a graph 61 of a change in free fatty acid concentration on an upper end of the display 60, and displaying detailed information 62 at a specific time point on a lower end thereof. As illustrated herein, a predetermined mark M may be superimposed on the graph 61 of the change in free fatty acid concentration, and when a user places the mark M on a time point (measurement index 5), for which a user wants to see detailed information, the display 60 may display the detailed information 62 at the corresponding time point on the lower end thereof.

Upon receiving a request for estimating bio-information, the processor 200 may guide a user on a measurement position of bio-impedance according to the type of bio-information to be estimated and/or impedance which may be measured by the impedance sensor 100. For example, in the case where the impedance sensor 100 is configured to measure the abdomen impedance, the processor 200 may guide the user to place the impedance sensor 100 on the stomach, and in the case where the impedance sensor 100 is configured to measure the partial impedance from the upper body/arm/leg, etc., the processor 200 may guide the user to place the impedance sensor 100 on the upper body/arm/leg and the like. In addition, in the case where the impedance sensor 100 is configured to measure both the abdomen impedance and the partial impedance from the whole body/stomach/upper body/arm/leg, etc., the processor 200 may guide the user to measure the impedance according to bio-information to be estimated, e.g., to measure the impedance from the stomach if the bio-information is free fatty acid/triglyceride levels, and to measure the impedance from the upper body/arm/leg if the bio-information is the blood test marker such as uric acid/creatinine.

In particular, the output interface 510 may output a whole body image or an upper body image on the display and may display a mark superimposed on the image and indicative of a position (e.g., stomach or arm) for measuring the impedance. Alternatively, the output interface 510 may output a text, such as "please place the impedance sensor on the stomach," on the display to guide the user on a body part to be measured such as the stomach/arm/leg/upper body and the like. Alternatively, the output interface 510 may output the text, such as "please place the impedance sensor on the stomach," by voice using the audio output module.

The storage 520 may store various data, such as data processed by the impedance sensor 100 and/or the processor 200, for example, the bio-information estimation model, the estimated bio-information value, the user characteristic information, the measurement time, and the like. The storage 520 may include at least one storage medium of a flash memory type memory, a hard disk type memory, a multimedia card micro type memory, a card type memory (e.g., an SD memory, an XD memory, etc.), a Random Access Memory (RAM), a Static Random Access Memory (SRAM), a Read Only Memory (ROM), an Electrically Erasable Programmable Read Only Memory (EEPROM), a Programmable Read Only Memory (PROM), a magnetic memory, a magnetic disk, and an optical disk, and the like, but is not limited thereto.

The communication interface 530 may communicate with an external device to transmit and receive data, related to estimating bio-information, to and from the external device. The external device may include an information processing device, such as a smartphone, a tablet PC, a desktop computer, a laptop computer, and the like. The communication interface 530 may communicate with the external device by using various wired or wireless communication techniques, such as Bluetooth communication, Bluetooth Low Energy (BLE) communication, Near Field Communication (NFC), WLAN communication, Zigbee communication, Infrared Data Association (IrDA) communication, Wi-Fi Direct (WFD) communication, Ultra-Wideband (UWB) communication, Ant+ communication, WIFI communication, Radio Frequency Identification (RFID) communication, 3G, 4G, and 5G communications, and the like. However, this is merely exemplary and is not intended to be limiting.

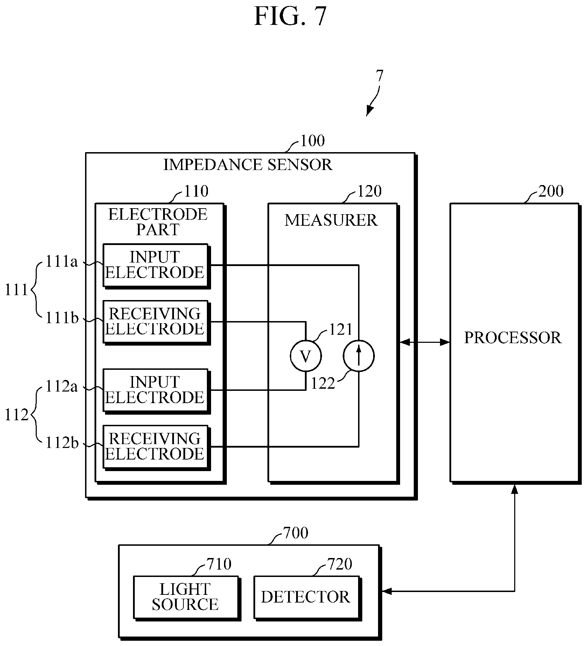

FIG. 7 is a block diagram illustrating an apparatus for estimating bio-information according to yet another example embodiment of the present disclosure.

Referring to FIG. 7, an apparatus 7 for estimating bio-information may include the impedance sensor 100, the processor 200, and an optical sensor 700, and may further include an output interface, a storage, and/or a communication interface. The impedance sensor 100 and the processor 200 are described in detail above, and thus the following description will be focused on non-redundant components and functions.

The optical sensor 700 may include a light source 710 for emitting light onto an object, and a detector 720 for detecting light returning from the object by being scattered or reflected from or transmitted into the object and the like after the light is emitted by the light source 710. The light source 710 may be formed as a light emitting diode (LED), a laser diode (LD), a phosphor, and the like. The light source 710 may be provided as an array which may emit light of different wavelengths, e.g., green, blue, red, and infrared wavelengths, and the like. The detector 720 may be formed as a photo diode, a photo transistor (PTr), an image sensor (e.g., Complementary Metal Oxide Semiconductor (CMOS) image sensor), etc., and may be provided as one or more arrays. The optical sensor 700 may be implemented as a spectrometer.

The processor 200 may estimate bio-information, such as free fatty acid, triglyceride, etc., by using the bio-impedance and the optical signal received from the impedance sensor 100 and the optical sensor 700, respectively. For example, the processor 200 may extract a feature from the optical signal, may combine the extracted feature with the bio-impedance to generate an estimation model for each bio-information to be estimated by using regression analysis and the like, and may estimate bio-information by using the generated estimation model. In particular, the processor 200 may extract an optimal feature predetermined for each bio-information to be estimated. Here, the feature may include, for example, heart rate, time and/or amplitude associated with propagation waves, time and/or amplitude associated with reflection waves, time and/or amplitude at a maximum amplitude point, time and/or amplitude at an internally dividing point between the time at the maximum amplitude point and the time associated with the propagation waves, total or partial area of a waveform of the optical signal, or a combination thereof.

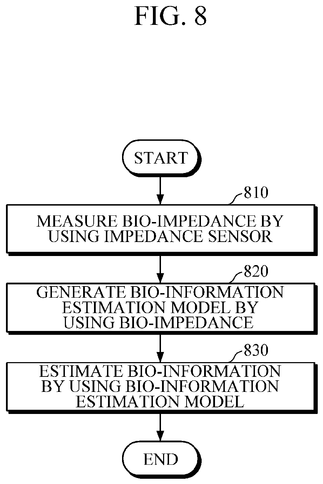

FIGS. 8 to 12 are flowcharts illustrating a method of estimating bio-information according to embodiments of the present disclosure.

The methods of FIGS. 8 to 12 are various embodiments performed by the apparatuses 1, 5, and 7 for estimating bio-information, which are described in detail above, and thus will be briefly described below.

Referring to FIG. 8, the apparatus for estimating bio-information measures bio-impedance from a user by using the impedance sensor in operation 810. Then, the apparatus for estimating bio-information generates an estimation model for estimating bio-information by analyzing a correlation between the measured bio-impedance and bio-information in operation 820. In particular, the bio-information may include free fatty acid, triglyceride, levels of accumulated/hydrolyzed/consumed fat/fat cells, calories that turn into energy, and/or blood test markers such as uric acid/creatinine, and the like. Next, when the bio-impedance is measured at a bio-information estimation time, the apparatus for estimating bio-information may estimate the bio-information in operation 830 based on the bio-impedance by using the bio-information estimation model generated in operation 820.

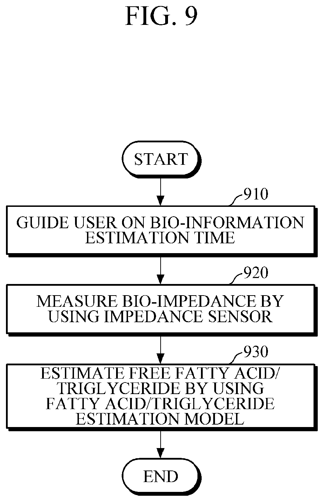

Referring to FIG. 9, the apparatus for estimating bio-information may guide a user to measure impedance at a predetermined bio-information estimation time in 910. For example, the bio-information estimation time may be predetermined according to bio-information monitoring purposes, such as continuous monitoring of a change in bio-information over a predetermined period of time in a fasting state, monitoring of a change in bio-information due to the effect of food intake, exercise, etc., monitoring of a change in bio-information during a day, and the like, and the apparatus for estimating bio-information may output guide information to a user at a measurement time by using the display, the audio output device, and/or the haptic device. Then, once the user measures the bio-impedance by using the impedance sensor according to the guide information at the measurement time in operation 920, the apparatus for estimating bio-information may estimate bio-information, such as free fatty acid, triglyceride, levels of accumulated/hydrolyzed/consumed fat, calories, and blood test markers, by using a pre-generated estimation model in operation 930. When estimating the bio-information is complete, the apparatus for estimating bio-information may output an estimated bio-information value. In particular, the apparatus for estimating bio-information may output a change in bio-information over a predetermined period of time, warning/alarm information, recommendations such as recommended exercise or food, and the like to a user.

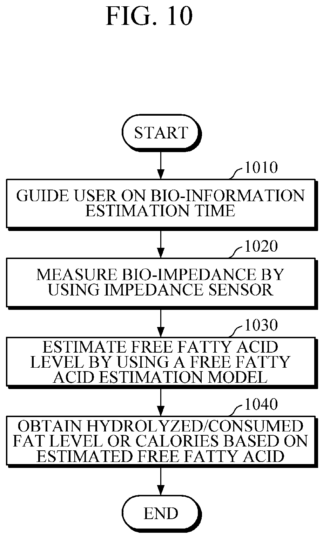

Referring to FIG. 10, the apparatus for estimating bio-information may guide a user to measure impedance at a predetermined bio-information estimation time in operation 1010, and once the user measures the bio-impedance by using the impedance sensor in operation 1020, the apparatus for estimating bio-information may estimate free fatty acid by using a free fatty acid estimation model in operation 1030. Subsequently, based on a correlation between a free fatty acid level and levels of accumulated/hydrolyzed/consumed fat or calories that turn into energy, the apparatus for estimating bio-information may obtain the levels of accumulated/hydrolyzed/consumed fat or the calories that turn into energy in operation 1040 based on the free fatty acid measured in operation 1030.

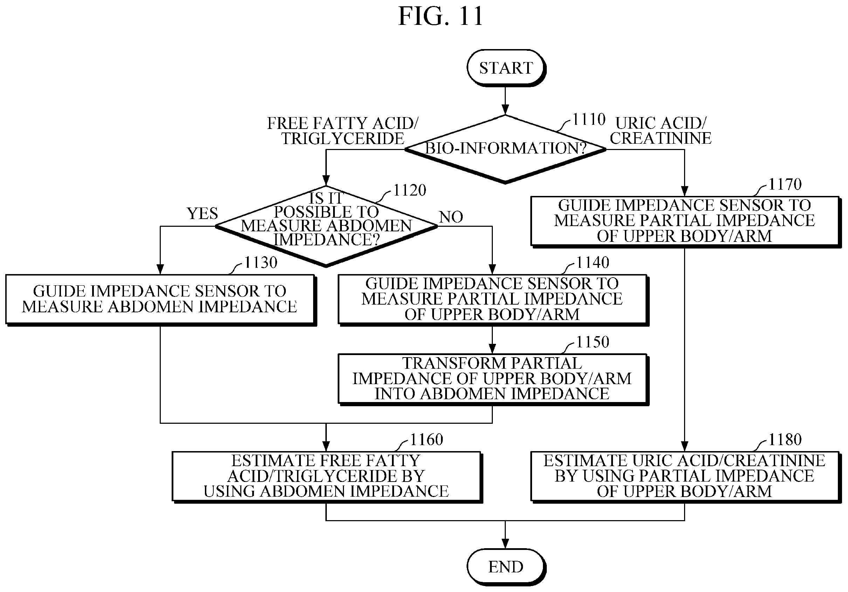

Referring to FIG. 11, upon receiving a request for estimating bio-information, the apparatus for estimating bio-information may check bio-information to be estimated in operation 1110. Upon checking in operation 1110, if the bio-information to be estimated is free fatty acid or triglyceride, the apparatus for estimating bio-information may determine whether the impedance sensor may measure the abdomen impedance in operation 1120. If the impedance sensor may measure the abdomen impedance, the apparatus for estimating bio-information may guide the impedance sensor to measure the abdomen impedance in operation 1130. Upon determination, if the impedance sensor is not capable of measuring the abdomen impedance, the apparatus for estimating bio-information may guide the impedance sensor to measure the partial impedance of the upper body/arm/leg and the like in operation 1140, and may transform the partial impedance, measured from the upper body/arm/leg and the like, into the abdomen impedance by using a transformation model in operation 1150. Next, the apparatus for estimating bio-information may estimate free fatty acid or triglyceride in operation 1160 by using the abdomen impedance measured in 1130 or the abdomen impedance transformed in 1150. Upon checking in operation 1110, if the bio-information to be estimated is uric acid/creatinine, the apparatus for estimating bio-information may guide the impedance sensor to measure the partial impedance of the upper body/arm/leg in operation 1170, and may estimate uric acid or creatinine by using the partial impedance in operation 1180.

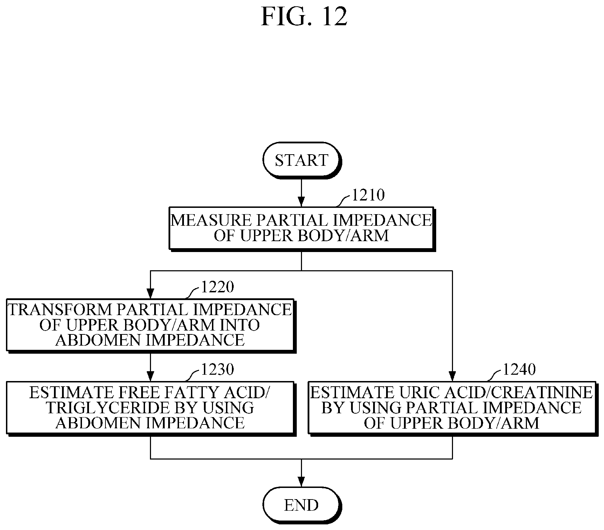

Referring to FIG. 12, if the impedance sensor is configured to measure only the partial impedance of the upper body/arm/leg and the like, the apparatus for estimating bio-information may guide the impedance sensor to measure the partial impedance of the upper body/arm/leg in operation 1210 immediately upon receiving the request for estimating bio-information, may transform the partial impedance into the abdomen impedance in operation 1220 to estimate free fatty acid or triglyceride by using the abdomen impedance in operation 1230, and may estimate uric acid/creatinine by using the partial impedance in operation 1240.

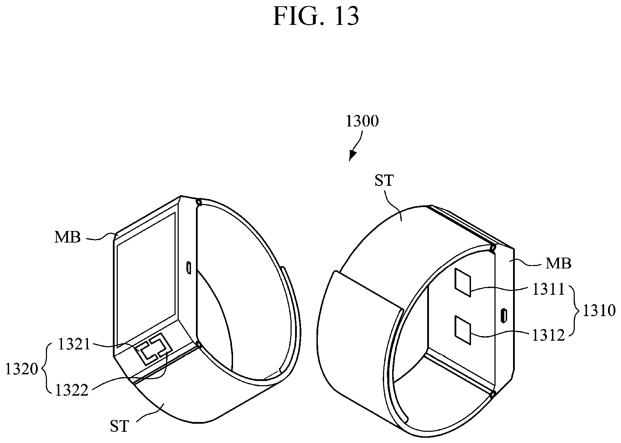

FIGS. 13 to 17 are diagrams schematically illustrating structures of various electronic devices including an apparatus for estimating bio-information.

As illustrated in FIGS. 13 to 17, the electronic device may be implemented as smart watch wearable devices 1300 and 1400, a band type wearable device 1500, a mobile device 1600 such as a smartphone, or an ear wearable device 1700, and the like. However, the electronic device is not limited thereto, and may include smart glasses, a smart ring, a smart patch, a smart necklace, a tablet PC, and the like. The electronic device may include the aforementioned apparatuses 1, 5, and 7 for estimating bio-information, and components of the apparatuses 1, 5, and 7 for estimating bio-information may be integrally formed in a single electronic device or may be separately mounted in two or more electronic devices.

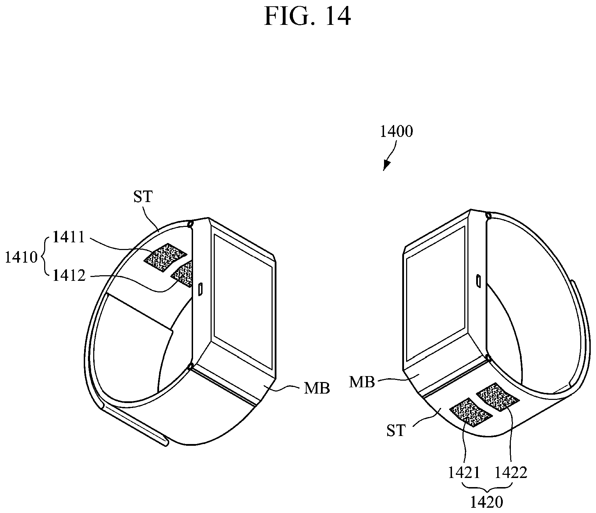

Referring to FIGS. 13 and 14, the electronic device may be implemented as the wristwatch wearable devices 1300 and 1400 and may include a main body MB and a wrist strap ST. A display may be provided on a front surface of the main body, and may display a general application screen displaying time information, received message information, etc., and/or a bio-information estimation application displaying guide information on a measurement time point, a bio-information estimation result, and the like.

The impedance sensor includes electrodes parts, in which a first electrode part 1310 including an input electrode 1311 and a receiving electrode 1312 may be disposed on a rear surface of the main body MB to come into contact with a user's wrist, and a second electrode part 1320 including an input electrode 1321 and a receiving electrode 1322 may be disposed on a front surface of the main body MB to come into contact with another body part of the user, e.g., a finger, as illustrated in FIG. 13. Alternatively, as illustrated in FIG. 14, a first electrode part 1410 including an input electrode 1411 and a receiving electrode 1412 may be disposed on an inner surface of the strap ST to come into contact with the wrist, and a second electrode part 1420 including an input electrode 1421 and a receiving electrode 1422 may be disposed on an outer surface of the strap ST to come into contact with another body part of the user, e.g., the finger. However, the present disclosure is not limited thereto, and one of the first electrode part and the second electrode part may be disposed on the main body MB, and the other one may be disposed on the strap ST.

A measurer of the impedance sensor, which is electrically connected to the electrode parts of the impedance sensor, a processor for estimating bio-information by using the bio-impedance, a storage for storing data related to estimating bio-information, an output interface for outputting data generated by the processor through a display and the like, and a communication interface for communicating with another electronic device, and the like may be included in the main body MB.

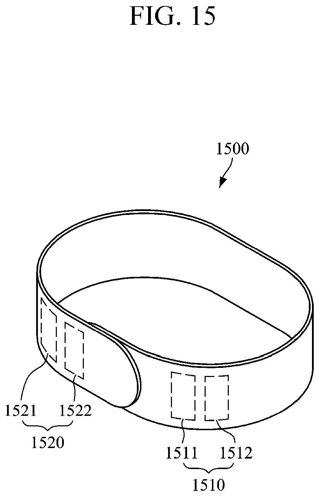

Referring to FIG. 15, the electronic device may be implemented as a band type wearable device 1500. As illustrated herein, the band type wearable device 1500 may include electrode parts 1510 and 1520 having input electrodes 1511 and 1521 and receiving electrodes 1512 and 1522 which are formed in the band to measure impedance from the stomach/arm/leg which comes into contact with the band type wearable device 1500 when the band type wearable device 1500 is worn thereon. A measurer, a processor, and/or a communication interface may be included in the band, and bio-information estimated by the processor may be transmitted to an external device through the communication interface to be output to a display of the external device.

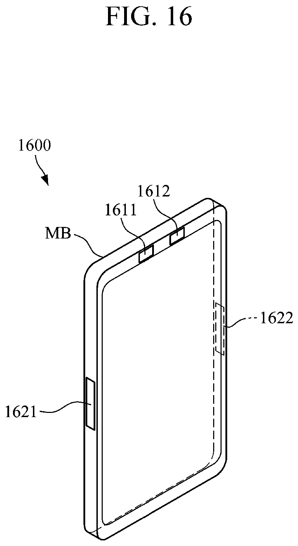

Referring to FIG. 16, the electronic device may be implemented as the mobile device 1600 such as a smartphone.

The mobile device 1600 may include a main body MB and a display panel. The main body MB may form an exterior of the mobile device 1600. The main body MB has a first surface, on which a display panel and a cover glass may be disposed sequentially, and the display panel may be exposed to the outside through the cover glass. A measurer, a processor, a communication interface, a storage, and the like of an impedance sensor may be disposed in the main body MB. The impedance sensor includes electrode parts, in which an input electrode 1611 and a receiving electrode 1612 of a first electrode part are disposed at a predetermined position of the main body MB, e.g., a front upper end coming into contact with the ear during a phone conversation. Alternatively, the electrodes may be provided as transparent electrodes on a front liquid crystal panel of the main body MB. Further, an input electrode 1621 and a receiving electrode 1622 of a second electrode part may be disposed at another position of the main body MB, e.g., both sides coming into contact with the hand during a phone conversation. Alternatively, the electrodes may be disposed on a rear surface of the main body MB to come into contact with the palm of the hand while a user holds the main body MB.

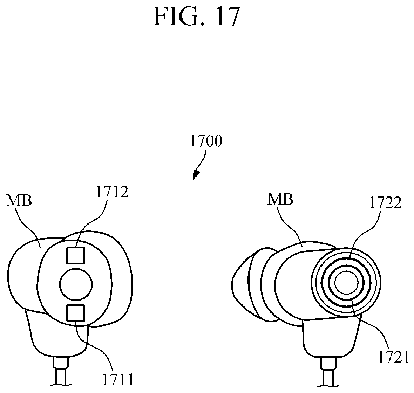

Referring to FIG. 17, the electronic device may be implemented as the ear wearable device 1700.

While listening to music with a main body MB of the ear wearable device 1700 being inserted into the ear, a user may measure bio-impedance by touching the main body MB with a finger. As illustrated herein, an input electrode 1711 and a receiving electrode 1712 of a first electrode part may be disposed on an inner surface of the main body MB so as to come into contact with the inside of the ear when the main body MB is inserted into the ear. In addition, an input electrode 1721 and a receiving electrode 1722 of a second electrode part may be disposed on an outer surface of the main body MB so as to come into contact with the finger while the main body MB is inserted into the ear. As illustrated here, the electrodes may be arranged in a concentric circle, but depending on the shape of the outer surface of the main body MB, the electrodes may be formed in a bar shape, a semicircular shape, a rectangular shape, and the like. A measurer, a processor, and/or a communication interface of the impedance sensor may be mounted in the main body MB, and may be connected by wire or wirelessly to an external device to transmit the measured bio-impedance to a mobile terminal or a wearable device so that the external device may estimate or output bio-information.

While not restricted thereto, an example embodiment can be embodied as computer-readable code on a computer-readable recording medium. The computer-readable recording medium is any data storage device that can store data that can be thereafter read by a computer system. Examples of the computer-readable recording medium include read-only memory (ROM), random-access memory (RAM), CD-ROMs, magnetic tapes, floppy disks, and optical data storage devices. The computer-readable recording medium can also be distributed over network-coupled computer systems so that the computer-readable code is stored and executed in a distributed fashion. Also, an example embodiment may be written as a computer program transmitted over a computer-readable transmission medium, such as a carrier wave, and received and implemented in general-use or special-purpose digital computers that execute the programs. Moreover, it is understood that in example embodiments, one or more units of the above-described apparatuses and devices can include circuitry, a processor, a microprocessor, etc., and may execute a computer program stored in a computer-readable medium.

Also, the description of the exemplary embodiments is intended to be illustrative, and not to limit the scope of the claims, which is defined by the appended claims.

## Claims

1. An apparatus for estimating bio-information, the apparatus comprising:
an impedance sensor (100) comprising a pair of input electrodes (111a, 112a) and a pair of receiving electrodes (111b, 112b), and configured to measure bio-impedance of a user by applying a current to the pair of input electrodes and by measuring a voltage between the pair of receiving electrodes; and
a processor (200) configured to estimate bio-information by applying, to the measured bio-impedance, an estimation model that uses a correlation between the measured bio-impedance and the bio-information to be estimated,
wherein the bio-information comprises free fatty acid or triglyceride, and also a blood test marker;
wherein the processor is further configured to generate user guidance information about a bio-impedance measurement position according to a type of the bio-information to be estimated, and wherein:
in response to the bio-information to be estimated being the free fatty acid or the triglyceride, the processor is further configured to control the impedance sensor to measure an abdomen impedance from a stomach of the user, wherein the processor is configured to guide the user to place the impedance sensor on the stomach of the user; and
in response to the bio-information to be estimated being the blood test marker, the processor is further configured to control the impedance sensor to measure a partial impedance from a torso, an arm, or a leg of the user, wherein the processor is configured to guide the user to place the impedance sensor on the torso, arm, or leg of the user.

2. The apparatus of claim 1, wherein the correlation comprises a correlation between the bio-impedance and a free fatty acid concentration in a tissue fluid.

3. The apparatus of claim 1, wherein the bio-information further comprises at least one of an accumulated fat level, a hydrolyzed fat level, a consumed fat level, and calories that have turned into energy.

4. The apparatus of claim 1, wherein the correlation comprises a correlation between the bio-impedance and a free fatty acid concentration in a tissue fluid, and
wherein the processor is further configured to estimate the bio-information by estimating the free fatty acid concentration in the tissue fluid by using the estimation model based on the correlation between the bio-impedance and the free fatty acid concentration in the tissue fluid; and estimating the free fatty acid concentration in blood from the free fatty acid concentration in the tissue fluid based on a correlation between the free fatty acid concentration in the tissue fluid and the free fatty acid concentration in blood.

5. The apparatus of one of claims 1 to 4, wherein the blood test marker comprises at least one of uric acid, creatinine, cholesterol, blood pressure, glycated hemoglobin, HbA1C, blood glucose, hematocrit, Hct, and plasma protein.

6. The apparatus of claim 5, wherein the processor is further configured to perform at least one of
generating at least one of exercise information or food intake information for guiding the user, based on an estimated triglyceride concentration or the blood test marker, and
generating the estimation model that uses regression analysis to estimate the blood test marker, based on at least one of a gender, an age, a stature, and a weight of the user.

7. The apparatus of one of claims 1 to 6, further comprising an output interface (510) configured to output information on an estimation time of the bio-information by using at least one of a display, an audio output device, and a haptic device, and wherein the output interface comprises the display configured to display a change in the bio-information over a predetermined period of time, or
further comprising an optical sensor (700) configured to emit light onto an object and to detect an optical signal scattered or reflected from the object, wherein the processor is further configured to generate the estimation model based on the bio-impedance and the detected optical signal.

8. A method of estimating bio-information, the method comprising:
measuring bio-impedance of a user by using an impedance sensor;
estimating bio-information by applying, to the measured bio-impedance, an estimation model that uses a correlation between the measured bio-impedance and the bio-information to be estimated,
wherein the bio-information comprises free fatty acid or triglyceride, and also a blood test marker;
generating user guidance information about a bio-impedance measurement position according to a type of the bio-information to be estimated; and
in response to the bio-information to be estimated being the free fatty acid or the triglyceride, controlling the impedance sensor to measure an abdomen impedance from a stomach of the user, wherein the user is guided to place the impedance sensor on the stomach of the user; and
in response to the bio-information to be estimated being the blood test marker, controlling the impedance sensor to measure a partial impedance from a torso, an arm, or a leg of the user, wherein the user is guided to place the impedance sensor on the torso, arm, or leg of the user.

9. The method of claim 8 adapted to operate the apparatus of one of claims 1 to 7.

10. A computer-readable medium having instructions that, when executed by a processor (200) of an apparatus for estimating bio-information having an impedance sensor (100) comprising a pair of input electrodes (111a, 112a) and a pair of receiving electrodes (111b, 112b), the impedance sensor being configured to measure bio-impedance of a user by applying a current to the pair of input electrodes and by measuring a voltage between the pair of receiving electrodes, cause the processor to:
measure bio-impedance of a user by using an impedance sensor;
estimate bio-information by applying, to the measured bio-impedance, an estimation model that uses a correlation between the measured bio-impedance and the bio-information to be estimated,
wherein the bio-information comprises at least one of free fatty acid, triglyceride, and a blood test marker,
generate user guidance information about a bio-impedance measurement position according to a type of the bio-information to be estimated; and
in response to the bio-information to be estimated being the free fatty acid or the triglyceride, control the impedance sensor to measure an abdomen impedance from a stomach of the user, wherein the user is guided to place the impedance sensor on the stomach of the user; and
in response to the bio-information to be estimated being the blood test marker, control the impedance sensor to measure a partial impedance from a torso, an arm, or a leg of the user, wherein the user is guided to place the impedance sensor on the torso, arm, or leg of the user.

11. The computer-readable medium of claim 10, wherein the processor is further caused to generate an estimation model by analyzing a correlation between the bio-impedance and the at least one of the accumulated fat level, the hydrolyzed fat level, the consumed fat level, and the calories that have turned into energy, and to obtain at least one of the accumulated fat level, the hydrolyzed fat level, the consumed fat level, and the calorie level that have turned into energy from the bio-impedance by using the generated estimation model.

12. An electronic device (1300, 1400) comprising:
a main body; and
the apparatus of one of claims 1 to 7,
wherein the pair of input electrodes is a first electrode pair that is disposed on a first surface of the main body to come into contact with a first body part of the user, the pair of receiving electrodes is a second electrode pair disposed on a second surface of the main body to come into contact with a second body part of the user, and
wherein the impedance sensor further comprises a measurer (120) configured to measure bio-impedance using the first electrode pair and the second electrode pair.

## Patentansprüche

1. Vorrichtung zum Schätzen von Bioinformation, wobei die Vorrichtung aufweist:
einen Impedanzsensor (100), der zwei als Paar vorgesehene Eingangselektroden (111a, 112a) und zwei als Paar vorgesehene Empfangselektroden (111b, 112b) aufweist und ausgebildet ist, die Bioimpedanz eines Benutzers zu messen, indem Strom in die beiden Eingangselektroden eingeprägt und eine Spannung zwischen den beiden Empfangselektroden gemessen wird; und
einen Prozessor (200), der ausgebildet ist, Bioinformation zu schätzen durch Anwenden eines Schätzmodells auf die gemessene Bioimpedanz, das eine Korrelation zwischen der gemessenen Bioimpedanz und der zu schätzenden Bioinformation verwendet, wobei die Bioinformation freie Fettsäuren oder Triglyceride sowie einen Bluttestmarker enthält;
wobei der Prozessor ferner ausgebildet ist, eine Benutzeranleitungsinformation über eine Bioimpedanzmessposition entsprechend einem Typ der zu schätzenden Bioinformation zu generieren, und wobei:
als Reaktion darauf, dass die zu schätzende Bioinformation die freie Fettsäure oder das Triglycerid ist, der Prozessor ferner ausgebildet ist, den Impedanzsensor so zu steuern, dass er eine Bauchimpedanz vom Magen des Benutzers misst, wobei der Prozessor ausgebildet ist, den Benutzer anzuleiten, den Impedanzsensor auf den Magen des Benutzers zu platzieren; und
als Reaktion darauf, dass die zu schätzende Bioinformation der Bluttestmarker ist, der Prozessor ferner ausgebildet ist, den Impedanzsensor so steuert, dass er eine Teilimpedanz aus einem Oberkörper, einem Arm oder einem Bein des Benutzers misst, wobei der Prozessor ausgebildet ist, den Benutzer anzuleiten, den Impedanzsensor auf dem Oberkörper, einem Arm oder Bein des Benutzers zu platzieren.

2. Vorrichtung nach Anspruch 1, wobei die Korrelation eine Korrelation zwischen der Bioimpedanz und einer freien Fettsäurekonzentration in einer Gewebeflüssigkeit umfasst.

3. Vorrichtung nach Anspruch 1, wobei die Bioinformation ferner mindestens einen der folgenden Werte umfasst: einen akkumulierten Fettgehalt, einen hydrolysierten Fettgehalt, einen verbrauchten Fettgehalt und Kalorien, die in Energie umgewandelt wurden.

4. Vorrichtung nach Anspruch 1, wobei die Korrelation eine Korrelation zwischen der Bioimpedanz und einer freien Fettsäurekonzentration in einer Gewebeflüssigkeit umfasst, und
wobei der Prozessor ferner ausgebildet ist, die Bioinformation zu schätzen durch Schätzen der freien Fettsäurekonzentration in der Gewebeflüssigkeit unter Verwendung des Schätzmodells auf der Grundlage der Korrelation zwischen der Bioimpedanz und der freien Fettsäurekonzentration in der Gewebeflüssigkeit; und durch Schätzen der freien Fettsäurekonzentration im Blut aus der freien Fettsäurekonzentration in der Gewebeflüssigkeit auf der Grundlage einer Korrelation zwischen der freien Fettsäurekonzentration in der Gewebeflüssigkeit und der freien Fettsäurekonzentration im Blut.

5. Vorrichtung nach einem der Ansprüche 1 bis 4, wobei der Bluttestmarker mindestens einen der folgenden Werte umfasst: Harnsäure, Kreatinin, Cholesterin, Blutdruck, glykiertes Hämoglobin, HbA1C, Blutzucker, Hämatokrit, Hct und Plasmaprotein.

6. Vorrichtung nach Anspruch 5, wobei der Prozessor ferner ausgebildet ist zum
Erzeugen mindestens einer Trainingsinformation oder einer Nahrungsaufnahmeinformation zur Anleitung des Benutzers auf der Grundlage einer geschätzten Triglyceridkonzentration oder des Bluttestmarkers, und/oder Erzeugen des Schätzmodells, das eine Regressionsanalyse verwendet, um den Bluttestmarker auf der Grundlage mindestens eines der folgenden Merkmale des Benutzers zu schätzen: Geschlecht, Alter, Statur und Gewicht.

7. Vorrichtung nach einem der Ansprüche 1 bis 6, die ferner eine Ausgabeschnittstelle (510) aufweist, die ausgebildet ist, Information über einen Schätzzeitpunkt der Bioinformation unter Verwendung mindestens einer Anzeige, einer Audioausgabevorrichtung und einer haptischen Vorrichtung auszugeben, und wobei die Ausgabeschnittstelle die Anzeige aufweist, die ausgebildet ist, eine Änderung der Bioinformation über einen vorbestimmten Zeitraum anzuzeigen, oder
Ferner einen optischen Sensor (700) aufweist, der ausgebildet ist, Licht auf ein Objekt auszusenden und ein von dem Objekt gestreutes oder reflektiertes optisches Signal zu erfassen, wobei der Prozessor ferner ausgebildet ist, das Schätzmodell auf der Grundlage der Bioimpedanz und des erfassten optischen Signals zu erzeugen.

8. Verfahren zum Schätzen von Bioinformation, wobei das Verfahren umfasst:
Messen der Bioimpedanz eines Benutzers unter Verwendung eines Impedanzsensors;
Schätzen von Bioinformation durch Anwenden eines Schätzmodells auf die gemessene Bioimpedanz, das eine Korrelation zwischen der gemessenen Bioimpedanz und der zu schätzenden Bioinformation verwendet, wobei die Bioinformation freie Fettsäuren oder Triglyceride sowie einen Bluttestmarker enthält;
Erzeugen von Benutzeranleitungsinformation über eine Bioimpedanzmessposition entsprechend einem Typ der zu schätzenden Bioinformation; und
als Reaktion darauf, dass die zu schätzende Bioinformation die freie Fettsäure oder das Triglycerid ist, Steuern des Impedanzsensors, um eine Bauchimpedanz vom Magen des Benutzers zu messen, wobei der Benutzer angeleitet wird, den Impedanzsensor auf dem Magen des Benutzers zu platzieren; und
als Reaktion darauf, dass die zu schätzende Bioinformation der Bluttestmarker ist, Steuern des Impedanzsensors, um eine Teilimpedanz vom Oberkörper, einem Arm oder einem Bein des Benutzers zu messen, wobei der Benutzer angeleitet wird, den Impedanzsensor auf dem Oberkörper, Arm oder Bein des Benutzers zu platzieren.

9. Verfahren nach Anspruch 8, das zum Betreiben der Vorrichtung nach einem der Ansprüche 1 bis 7 ausgebildet ist.

10. Computerlesbares Medium mit Anweisungen, die, wenn sie von einem Prozessor (200) einer Vorrichtung zum Schätzen von Bioinformation mit einem Impedanzsensor (100) ausgeführt werden, der zwei als Paar vorgesehene Eingangselektroden (111a, 112a) und zwei als Paar vorgesehene Empfangselektroden (111b, 112b) aufweist, wobei der Impedanzsensor ausgebildet ist, die Bioimpedanz eines Benutzers zu messen durch einprägen eines Stroms in die beiden Eingangselektroden und Messen einer Spannung zwischen den beiden Empfangselektroden, den Prozessor veranlassen zum:
Messen der Bioimpedanz eines Benutzers unter Verwendung eines Impedanzsensors;
Schätzen der Bioinformation durch Anwenden eines Schätzmodells auf die gemessene Bioimpedanz, das eine Korrelation zwischen der gemessenen Bioimpedanz und der zu schätzenden Bioinformation verwendet, wobei die Bioinformation freien Fettsäuren und/oder Triglyceride und/oder einem Bluttestmarker umfassen,
Erzeugen von Benutzeranleitungen bezüglich einer Bioimpedanz-Messposition entsprechend einem Typ der zu schätzenden Bioinformation; und
als Reaktion darauf, dass die zu schätzende Bioinformation die freie Fettsäure oder das Triglycerid ist, Steuern des Impedanzsensors derart, dass eine Bauchimpedanz vom Magen des Benutzers gemessen wird, wobei der Benutzer angeleitet wird, den Impedanzsensor auf den Magen des Benutzers zu platzieren; und
als Reaktion darauf, dass die zu schätzende Bioinformation der Bluttestmarker ist, Steuern des Impedanzsensors derart, dass eine Teilimpedanz vom Oberkörper, einem Arm oder einem Bein des Benutzers gemessen wird, wobei der Benutzer angeleitet wird, den Impedanzsensor auf dem Oberkörper, dem Arm oder dem Bein des Benutzers zu platzieren.

11. Computerlesbares Medium nach Anspruch 10, wobei der Prozessor ferner veranlasst wird zum
Erzeugen eines Schätzmodells durch Analysieren einer Korrelation zwischen der Bioimpedanz und dem angesammelten Fettanteil und/oder dem hydrolysierten Fettanteil und/oder dem verbrauchten Fettanteil und/oder den in Energie umgewandelten Kalorien, und Erhalten des angesammelten Fettanteils und/oder des hydrolysierten Fettanteils und/oder des verbrauchten Fettanteils und/oder des Kalorienanteils, der in Energie umgewandelt wurde, aus der Bioimpedanz unter Verwendung des erzeugten Schätzmodells.

12. Elektronisches Gerät (1300, 1400) mit:
einem Hauptkörper; und
der Vorrichtung nach einem der Ansprüche 1 bis 7,
wobei die beiden Eingangselektroden ein erstes Elektrodenpaar sind, das auf einer ersten Oberfläche des Hauptkörpers angeordnet ist, um mit einem ersten Körperteil des Benutzers in Kontakt zu kommen, die beiden Empfangselektroden ein zweites Elektrodenpaar sind, das auf einer zweiten Oberfläche des Hauptkörpers angeordnet ist, um mit einem zweiten Körperteil des Benutzers in Kontakt zu kommen, und
wobei der Impedanzsensor ferner eine Messeinrichtung (120) aufweist, die ausgebildet ist, die Bioimpedanz unter Verwendung des ersten Elektrodenpaares und des zweiten Elektrodenpaares zu messen.

## Revendications

1. Appareil d'estimation d'informations biologiques, l'appareil comprenant :
un capteur d'impédance (100) comprenant une paire d'électrodes d'entrée (111a, 112a) et une paire d'électrodes de réception (111b, 112b), et configuré pour mesurer la bio-impédance d'un utilisateur en appliquant un courant à la paire d'électrodes d'entrée et en mesurant une tension entre la paire d'électrodes de réception ; et
un processeur (200) configuré pour estimer des informations biologiques en appliquant, à la bio-impédance mesurée, un modèle d'estimation qui utilise une corrélation entre la bio-impédance mesurée et les informations biologiques à estimer,
dans lequel les informations biologiques comprennent un acide gras libre ou un triglycéride, et également un marqueur de test sanguin ;
dans lequel le processeur est en outre configuré pour générer des informations de guidage d'utilisateur concernant une position de mesure de bio-impédance selon un type d'informations biologiques à estimer, et dans lequel :
en réponse au fait que les informations biologiques à estimer sont l'acide gras libre ou le triglycéride, le processeur est en outre configuré pour commander le capteur d'impédance afin de mesurer une impédance abdominale à partir de l'abdomen de l'utilisateur, dans lequel le processeur est configuré pour guider l'utilisateur afin de placer le capteur d'impédance sur l'abdomen de l'utilisateur ; et
en réponse au fait que les informations biologiques à estimer sont le marqueur de test sanguin, le processeur est en outre configuré pour commander le capteur d'impédance afin de mesurer une impédance partielle à partir du torse, d'un bras ou d'une jambe de l'utilisateur, dans lequel le processeur est configuré pour guider l'utilisateur afin de placer le capteur d'impédance sur le torse, le bras ou la jambe de l'utilisateur.

2. Appareil selon la revendication 1, dans lequel la corrélation comprend une corrélation entre la bio-impédance et une concentration en acides gras libres dans un fluide tissulaire.

3. Appareil selon la revendication 1, dans lequel les informations biologiques comprennent en outre au moins l'un parmi un niveau de graisse accumulée, un niveau de graisse hydrolysée, un niveau de graisse consommée et des calories qui se sont transformées en énergie.

4. Appareil selon la revendication 1, dans lequel la corrélation comprend une corrélation entre la bio-impédance et une concentration en acides gras libres dans un fluide tissulaire, et
dans lequel le processeur est en outre configuré pour estimer les informations biologiques en estimant la concentration en acides gras libres dans le fluide tissulaire par utilisation du modèle d'estimation sur la base de la corrélation entre la bio-impédance et la concentration en acides gras libres dans le fluide tissulaire ; et en estimant la concentration en acides gras libres dans le sang à partir de la concentration en acides gras libres dans le liquide tissulaire sur la base d'une corrélation entre la concentration en acides gras libres dans le liquide tissulaire et la concentration en acides gras libres dans le sang.

5. Appareil selon l'une des revendications 1 à 4, dans lequel le marqueur de test sanguin comprend au moins l'un parmi l'acide urique, la créatinine, le cholestérol, la pression artérielle, l'hémoglobine glyquée, l'HbA1C, la glycémie, l'hématocrite, l'Hct et la protéine plasmatique.

6. Appareil selon la revendication 5, dans lequel le processeur est en outre configuré pour effectuer au moins l'une parmi
la génération des informations sur l'exercice physique et/ou des informations sur l'apport alimentaire pour guider l'utilisateur, sur la base d'une concentration estimée en triglycérides ou du marqueur de test sanguin, et
la génération du modèle d'estimation qui utilise une analyse de régression pour estimer le marqueur de test sanguin, sur la base d'au moins l'un parmi le sexe, l'âge, la taille et le poids de l'utilisateur.

7. Appareil selon l'une des revendications 1 à 6, comprenant en outre une interface de sortie (510) configurée pour délivrer en sortie des informations sur un temps d'estimation des informations biologiques en utilisant au moins l'un parmi un écran, un dispositif de sortie audio et un dispositif haptique, et dans lequel l'interface de sortie comprend l'écran configuré pour afficher un changement dans les informations biologiques sur une période de temps prédéterminée, ou
comprenant en outre un capteur optique (700) configuré pour émettre de la lumière sur un objet et pour détecter un signal optique diffusé ou réfléchi par l'objet, dans lequel le processeur est en outre configuré pour générer le modèle d'estimation sur la base de la bio-impédance et du signal optique détecté.

8. Procédé d'estimation d'informations biologiques, le procédé comprenant :
la mesure de la bio-impédance d'un utilisateur en utilisant un capteur d'impédance ;
l'estimation d'informations biologiques en appliquant, à la bio-impédance mesurée, un modèle d'estimation qui utilise une corrélation entre la bio-impédance mesurée et les informations biologiques à estimer,
dans lequel les informations biologiques comprennent un acide gras libre ou un triglycéride, et également un marqueur de test sanguin ;
la génération d'informations de guidage d'utilisateur concernant une position de mesure de bio-impédance selon un type d'informations biologiques à estimer ; et
en réponse au fait que les informations biologiques à estimer sont l'acide gras libre ou le triglycéride, la commande du capteur d'impédance pour mesurer une impédance abdominale à partir de l'abdomen de l'utilisateur, dans lequel l'utilisateur est guidé pour placer le capteur d'impédance sur l'abdomen de l'utilisateur ; et
en réponse au fait que les informations biologiques à estimer sont le marqueur de test sanguin, la commande du capteur d'impédance pour mesurer une impédance partielle à partir du torse, d'un bras ou d'une jambe de l'utilisateur, dans lequel l'utilisateur est guidé pour placer le capteur d'impédance sur le torse, le bras ou la jambe de l'utilisateur.

9. Procédé selon la revendication 8 adapté pour faire fonctionner l'appareil selon l'une des revendications 1 à 7.

10. Support lisible par ordinateur ayant des instructions qui, lorsqu'elles sont exécutées par un processeur (200) d'un appareil d'estimation d'informations biologiques ayant un capteur d'impédance (100) comprenant une paire d'électrodes d'entrée (111 a, 112a) et une paire d'électrodes de réception (111b, 112b), le capteur d'impédance étant configuré pour mesurer la bio-impédance d'un utilisateur en appliquant un courant à la paire d'électrodes d'entrée et en mesurant une tension entre la paire d'électrodes de réception, amènent le processeur à :
mesurer la bio-impédance d'un utilisateur en utilisant un capteur d'impédance ;
estimer des informations biologiques en appliquant, à la bio-impédance mesurée, un modèle d'estimation qui utilise une corrélation entre la bio-impédance mesurée et les informations biologiques à estimer,
dans lequel les informations biologiques comprennent au moins l'un parmi un acide gras libre, un triglycéride et un marqueur de test sanguin,
générer des informations de guidage d'utilisateur concernant une position de mesure de bio-impédance selon un type d'informations biologiques à estimer ; et
en réponse au fait que les informations biologiques à estimer sont l'acide gras libre ou le triglycéride, commander le capteur d'impédance pour mesurer l'impédance abdominale à partir de l'abdomen de l'utilisateur, dans lequel l'utilisateur est guidé pour placer le capteur d'impédance sur l'abdomen de l'utilisateur ; et
en réponse au fait que les informations biologiques à estimer sont le marqueur de test sanguin, commander le capteur d'impédance pour mesurer une impédance partielle à partir du torse, d'un bras ou d'une jambe de l'utilisateur, dans lequel l'utilisateur est guidé pour placer le capteur d'impédance sur le torse, le bras ou la jambe de l'utilisateur.

11. Support lisible par ordinateur selon la revendication 10, dans lequel le processeur est en outre amené à générer un modèle d'estimation en analysant une corrélation entre la bio-impédance et l'au moins un parmi le niveau de graisse accumulée, le niveau de graisse hydrolysée, le niveau de graisse consommée et les calories qui ont été transformées en énergie, et à obtenir au moins l'un parmi le niveau de graisse accumulée, le niveau de graisse hydrolysée, le niveau de graisse consommée et le niveau de calories qui ont été transformées en énergie à partir de la bio-impédance en utilisant le modèle d'estimation généré.

12. Dispositif électronique (1300, 1400) comprenant :
un corps principal ; et
l'appareil selon l'une des revendications 1 à 7,
dans lequel la paire d'électrodes d'entrée est une première paire d'électrodes qui est disposée sur une première surface du corps principal pour entrer en contact avec une première partie de corps de l'utilisateur, la paire d'électrodes de réception est une seconde paire d'électrodes disposée sur une deuxième surface du corps principal pour entrer en contact avec une seconde partie de corps de l'utilisateur, et
dans lequel le capteur d'impédance comprend en outre un dispositif de mesure (120) configuré pour mesurer la bio-impédance en utilisant la première paire d'électrodes et la seconde paire d'électrodes.
